# EUROPEAN PATENT APPLICATION

(11) **EP 1 655 344 A1**
(43) Date of publication of application: **10.05.2006**
(21) Application number: 04026243.8
(22) Date of filing: 05.11.2004
(51) Int. Cl.: C08L 77/00, C08L 77/02, C08L 77/06, C08L 75/04, C08L 67/02, A61L 9/04

(54) **Polymeric compositions for sustained release of volatile materials**

(71) Applicant: THE PROCTER & GAMBLE COMPANY, Cincinnati, Ohio 45202 (US)
(72) Inventor: Corzani, Italo, 66100 Chieti (IT); Mariani, Manuel, 65015 Montesilvao (Pescara) (IT); MacBeath, Calum, 66023 Francavilla al Mare (Chieti) (IT); Rosati, Rodrigo, 66023 Francavilla al Mare (Chieti) (IT)
(74) Representative: Veronese, Pancrazio

(57) **Abstract**

Polymeric compositions which are able to incorporate and sustainedly release volatile materials (e.g. perfumes) based on the combined use of low melting point polyamide polymers with a polar thermoplastic elastomer. Such compositions are easy to process at low temperature and are able to stably incorporate and gradually release large amounts of volatile material.

## Description

### Field of the Invention

The present invention relates to polymeric compositions which are able to incorporate and sustainedly release volatile materials (e.g. perfumes) based on the combined use of low melting point polyamide polymers with polar thermoplastic elastomer. The compositions of the present invention can find a variety of applications wherein a prolonged delivery of a volatile material in an environment is desired such as air freshening devices, deodorants, scented objects, insecticides etc.

### Background of the Invention

Polymeric compositions which are able to absorb and release volatile ingredients are well known in the art, in particular concerning perfume delivery.

GB1558960 from Nagae, describes a perfume emitting PVC film to be used in umbrellas.

US 4618629 from T. Burnett & Co, Inc describes fragrance-emitting polyurethane foams which have a particulate fragrance-carrying resin incorporated in them. The resin can be selected from a list of polymers (polyolefins, polyester, PVC and similar, polyamides, cellulose polymers).

A common use of polymeric compositions for perfume delivery comprises for example air freshening devices. These are typically in the form of aqueous gels usually obtained from crosslinked polysaccharide polymers (starches, alginates or CMC) such as those described in GB2286531 from Kelco, US3969280 from Johnson & Johnson.

While these and other documents claim to provide long lasting delivery of volatile materials, they are still far from being fully satisfactory for a number of reasons. These polymeric compositions can normally incorporate and release a very limited amount of volatile material, in most cases not exceeding 10% of the total weight of the compositions.
Furthermore, the above mentioned polymeric compositions are commonly used to deliver simple perfumes, typically consisting of a single volatile substance such as citronellol as they are simply not capable to consistently deliver a more sophisticated perfume as is increasingly desired by the modem perfume industry.

US 4734278 describes shaped bodies of block polyether -amide based resins (e.g. Pebax™) that provide sustained release of volatile actives (perfumes, deodorants, insecticides etc). An improvement has been obtained by Atochem who in WO 9726020A1 describe improved fragrant resins made by Pebax™ plus a complex perfume (i.e. more than 5 components). Such resins are able to deliver a complex perfume with a reduced separation of the volatile ingredients over time.

US4552693 describes transparent fragrance-emitting articles obtained from compositions comprising a thermoplastic polyamide resin, a plasticizer/solvent system comprising a sulfonamide plasticizer, and a fragrance. The advantage of using a plasticizer in these compositions is the possibility of processing said compositions (molding, extruding, filming) at relatively low temperatures.

Polymeric compositions based on low melting point polyamides are particularly desirable as they can be molded at relatively low temperatures so that minimal amounts of the volatile substance to be incorporated are lost during the molding process, and this without the need for an additonal plasticizer, furthermore said low melting point polyamides are desirable as they are able to incorporate and deliver a wide range of volatile materials. However compositions based on low melting point polyamides and comprising a high load of perfume (>30%) tend to gradually lose the solubilised perfume which migrates in liquid form on the surface of the polymeric material itself. This phenomenon is known as bleeding. Another drawback of the polymeric compositions based on low melting point polyamides is that said compositions tend to have a sharp decrease of the melting temperature when the concentration of the solubilised volatile material increases over a certain level. When this happens the material can become gel-like or waxy and lose its integrity even at room temperature or slightly above.

Therefore there is still a need for a polymeric material based on low melting point polyamides which is able to incorporate and sustainedly release a high load of volatile materials, which is capable of being easily processed and formed into an article and which is physically stable in the solid state at temperatures in a wide range around the room temperature (0-50°C).

### Summary of the Invention

The present invention relates to a polymeric composition comprising:
a) a low melting point polyamide polymer
b) a polar thermoplastic elastomer
c) a volatile material.

### Detailed Description of the Invention

It was surprisingly found that compositions comprising a) a low melting point polyamide polymer b) a polar thermoplastic elastomer and c) a volatile material, while being still moldable at relatively low temperatures (below 130°C, preferably below 110°C, more preferably below 100°C), are able to incorporate a high load of volatile material without bleeding and to maintain their integrity in the solid state in a wide range of temperatures around room temperature (0-50°C) so that articles made with the polymeric composition of the present invention can be used in different environments e.g for air fresheners or insect repellents to be used also in very hot countries.

Without being bound to any theory, it is believed that the polymeric matrix obtained by combining a polar thermoplastic elastomer and a low melting point polyamide polymer, maintains the ability of the polyamide to solubilize a broad range of volatile materials, and at the same time has improved mechanical properties and physical stability due to the polymer framework provided by the polar thermoplastic elastomer.

In the preferred case when the polar thermoplastic elastomer is a block copolymer comprising a polyamide block it is believed that the particular affinity between the polyamide block and the low melting point polyamide provides the polymeric matrix with further enhanced physical stability.

Another very important benefit provided by the polymeric compositions of the present invention is the possibility to introduce a wide range of volatile materials.

Prior art solutions described polymeric compositions based on specific polymers like polyether-polyamide block copolymers or pure polyamide polymers. As a consequence the choice of the volatile material was limited to those ingredients which were soluble or compatible with that specific polymer.

Differently from prior art, the compositions of the present invention are much more flexible in terms of the compositions of the volatile material which can be delivered, since the formulator can choose the first polymer among all low melting point polyamide polymers, and the second polymer among all polar thermoplastic elastomers. Additionally optional ingredients as a plasticizer and a number of other additives can be introduced into the formulation as explained in detail below to fine-tune its polarity characteristics. Such a formulation flexibility for the polymeric matrix (two different polymers, optionally plasticizers and other polymers or additives) allows the tuning of its polarity characteristics very precisely. This makes it possible to maximize the compatibility with any volatile material which could be introduced in the polymeric matrix thus obtaining a polymeric composition according to the present invention. Without being bound to any theory, it is believed that a certain polarity match between the polymeric matrix and the volatile material is required to provide good incorporation and sustained delivery of the volatile material.

Hence the low melting point polyamide polymer, the polar thermoplastic elastomer and possibly the additional optional ingredients of the polymeric compositions of the present invention can be selected such that the polarity of the polymeric matrix substantially matches the polarity of the volatile material, wherein the polarities can be evaluated with one of the methods known in the art such as by measuring their respective water/octanol partition coefficient.

The term "low melting point polyamide polymers" includes all polyamides having a melting point below 130°C, preferably below 110°C, more preferably below 100°C. Typically and preferably, the low melting point polyamides of the present invention are solid at room temperature. Preferred polyamides are terminated polyamides, particularly preferred are ester terminated polyamides. Examples of these low melting point polyamides include those marketed by Arizona chemicals under the trade name of Sylvaclear.

The term "polar thermoplastic elastomer" includes multiphase polymers that comprise "hard" and "soft" phases chemically bonded together in the polymer chain. The "hard" phase is solid at room temperature and flows upon heating. Examples include blocks of amide, ester and urethane groups. The "soft" phase is rubbery at room temperature. Examples include polyether blocks such as poly(ethylene glycol), poly(propylene glycol) or poly(tetramethylene glycol). At room temperature, the presence of the "hard" phases in the polymer imparts strength and good mechanical properties. When the polymer is heated, these phases become liquid and the polymer melts, allowing for processing in the molten state. Upon recooling to room temperature, the phases solidify and the good mechanical properties are regained. A comprehensive definition of thermoplastic elastomers can be found in Vol 9 of the Kirk-Othmer Encyclopedia of Chemical Technology (4th Edition - Wiley- Interscience, 1996) - under the voice "Elastomers", subvoice "Thermoplastic Elastomers".

Among these polymers those which are suitable for the present invention are those comprising at least one polar monomer. Polar monomers are those monomers which comprise at least a C-X linkage in the molecule wherein said C-X linkage is a polar linkage. Preferably X is an N, S, F, Cl or O atom. More preferably said polar linkage is part of a carbonyl group and, more preferably, of an ester group. Preferred polar monomers for the present invention are vinyl acetate, vinyl alcohol, methyl acrylate, ethyl acrylate, butyl acrylate, acrylic acid and salts formed therefrom, methacrylic acid and salts formed therefrom, maleic anhydride, glycidyl methacrylate and carbon monoxide. More preferably the hard phases preferably comprise blocks of amide, ester or urethane groups and the soft phases preferably comprise polyether blocks.

Examples of these polar thermoplastic elastomers include thermoplastic polyurethanes, such as those produced under the trade names ESTANE by Noveon, and PELLETHANE by Dow Chemicals; thermoplastic polyesters, also known as polyether ester copolymers, such as those produced under the trade names HYTREL by Dupont and ARNITEL by DSM, and thermoplastic polyamides, also known as polyether amide copolymers, such as those produced under the trade name PEBAX by Atofina.

The third essential component of the present invention is a volatile material which is incorporated and then sustainedly delivered by the compositions of the present invention.

Volatile materials which can be used in the present invention are for example flavors, deodorants, insecticides, pheromones, aromas, repelling agents and most advantageously, perfumes.

The benefits provided by the present invention are particularly relevant when the volatile material is a perfume. Perfumes are typically composed of many components of different volatility. The present invention, avoiding separation of the components based on their different volatility, allows the sustained delivery of the full perfume bouquet for a long time. In a preferred embodiment of the present invention the volatile material is a perfume which is preferably composed by a plurality of components, more preferably by more than 5 components.

As used herein the term perfume means any odoriferous material. In general, such materials are characterised by a vapour pressure less than atmospheric pressure at room temperatures. The perfumes employed herein will most often be liquid at room temperatures, but also can be solid such as the various camphoraceous perfumes known in the art. A wide variety of chemicals are known for perfumery uses, including materials such as aldehydes, ketones, esters, alcohols, terpenes and the like. Naturally occurring plant and animal oils and exudates comprising complex mixtures of various chemical components are known for use as perfumes, and such materials can be used herein. The perfumes herein can be relatively simple in their composition or can comprise highly sophisticated, complex mixtures of natural and synthetic chemical components, all chosen to provide any desired odor.

Typical perfumes which can be used in the present invention comprise, for example, woody/earthy bases containing exotic materials such as sandalwood oil, civet, patchouli oil and the like. Other suitable perfumes are for example light, floral fragrances, e.g., rose extract, violet extract and the like. Perfumes can be formulated to provide desirable fruity odours, e.g., lime, lemon, orange and the like.

In short, any chemically compatible material which emanates a pleasant or otherwise desirable odour can be used as a perfume in the present invention.

Perfume materials are described more fully in S. Arctander, Perfume Flavors and Chemicals. Vols. I and II. Aurthor, Montclair, N.J., and the Merck Index, 8th Edition, Merck & Co., Inc. Rahway, N.J.

Preferably the volatile material of the present invention is introduced in the polymeric composition in a form which does not prevent the chemicals which constitute said volatile material from being chemically dissolved in the polymeric matrix. In particular encapsulated volatile materials and chemicals which comprise volatile species covalently bonded to a non volatile one (e.g. pro-perfumes) are not recommended and preferably excluded for use herein as volatile materials according to the present invention. Without being bound to any theory, it is believed that the advantageous properties of the polymeric compositions of the present invention can be seen when the volatile material is solubilized in the plasticized polymeric matrix, as the volatile material release is linked to molecular level interaction between the volatile material and the plasticized polymer matrix.

Preferably the polymeric composition of the present invention comprises from 5% to 40%, preferably from 7% to 30%, more preferably from 15% to 25%, by weight of the polymeric composition, of the low melting point polyamide; from 2% to 30%, preferably from 5% to 20%, more preferably from 7% to 15%, by weight of the polymeric composition, of the polar thermoplastic elastomer, and from 30% to 90%, preferably from 40% to 80%, more preferably from 50% to 75%, by weight of the polymeric composition, of the volatile material.

The polymeric compositions of the present invention may in addition comprise additional optional components to further improve the processability of the compositions and also the mechanical characteristics as well as other characteristics as tackiness, resistance to ageing by light, oxygen and heat, visual appearance etc., of the objects formed from such polymeric compositions.

A preferred optional component in the polymeric compositions of the present invention is a plasticizer. The plasticizer is preferably present in the polymeric compositions of the present invention at a level from 0% to 60%, preferably from 5% to 40%, more preferably from 7% to 25%, by weight of the polymeric composition. Suitable plasticizers for use in the polymeric compositions according to the present invention include citric acid esters, low molecular weight polyesters, polyethers, liquid rosin esters, aromatic sulfonamides, phthalates, benzoates, sucrose esters, derivatives of polyfunctional alcohols (where polyfunctional means having 2 or more hydroxyl groups), adipates, tartrates, sebacates, esters of phosphoric acid, fatty acids and diacids, fatty alcohols and diols, epoxidised vegetable oils etc, and mixtures thereof. As already mentioned above, the different polarity of the different compatible plasticizers can be used to tune the polarity of the polymeric matrix in order to provide a better match with the polarity of the volatile material (wherein the polarities are measurable with any method known to those skilled in the art, for example water/octanol partition coefficient).

Other optional components include other copolymers that can be included in the formulations to improve their properties for example to increase adhesion or compatibility with substrates. To this purpose preferred optional copolymers are those featuring both polar and non-polar groups, for example: copolymers of ethylene and at least one other vinyl or acrylic monomer, copolymers of styrene and at least one other vinyl or acrylic monomer, copolymers of poly(vinyl alcohol), polyamides, copolymers of poly(vinyl pyrrolidone), polyacrylates, copolymers of polyvinyl ethers), ionomers, polyester amide copolymers, etc.

Other optional components which can be preferably used when the polymeric composition according to the present invention is a thermoplastic composition and preferably has a hot melt rheology are tackifying resins such as rosin derivatives, aliphatic resins, aromatic resins or mixed aliphatic-aromatic resins. The compositions of the present invention can be then formulated in order to also have the characteristics of a hot melt adhesive, in addition to the capability of releasing volatile materials. Further optional ingredients such as other polymers or copolymers, fillers, crosslinkers, pigments, dyes, antioxidants and other stabilizers, etc can also be added to provide desired properties to the composition.

The polymeric compositions of the present invention preferably are thermoplastic polymeric compositions. These can be manufactured by using any known process for manufacturing thermoplastic polymeric compositions and will typically comprise the steps of melting the polymer and then blending the plasticizer and the volatile material to form a homogeneous mass that is then cooled to obtain the polymeric composition according to the present invention. Among thermoplastic compositions preferred are those which have low melt temperature and viscosity and therefore are processable as hot melts. In these systems the loss of volatile material upon blending is minimized.

The polymeric compositions of the present invention may also be prepared using a polymer solution, either as an intermediate or final step. Preparations of this type are well known to those skilled in the art and typically will comprise the steps of dissolving the selected polymers, and volatile material in an effective solvent, and heating if necessary to prepare a solution or a gel. The solvent can then be eliminated by evaporation.

Alternatively, the polymeric compositions of the present invention can be prepared in the form of an aqueous emulsion or dispersion. The techniques for obtaining aqueous emulsions or dispersions of polymers are well known to the skilled man. For example, the selected polymers, and volatile material can be blended together as a thermoplastic material. The resulting melt can then be dispersed in water, preferably at a temperature above its melting point, by mixing. Surfactant and/or stabilizing systems known to those skilled in the art can be employed to stabilize the resultant emulsion or dispersion.

Alternatively, a preformed aqueous polymeric dispersion or emulsion can be blended with the selected volatile material. This can be done by adding the ingredients directly to the polymeric dispersion or emulsion, or by forming an aqueous dispersion of the perfume and blending this with the polymeric dispersion or emulsion. Both procedures result in the formation of an aqueous dispersion of a polymeric composition according to the present invention.

Alternatively, one of the polymers can be directly formed in a water dispersion in the presence of the volatile material and of the other polymer. This process can involve the solution or dispersion of monomers or prepolymers in water containing the dispersed volatile material and the other polymer. The polymerization can then be initiated to form the polymeric dispersion. If required, the volatile material can be alternatively added subsequently to produce a dispersed polymeric composition according to the present invention.

Polymeric compositions according to the present invention may have different applications whenever the release of a volatile material is desired. For example they can be used in air-freshening devices (room-fresheners, car fresheners, toilet rim-blocks etc.), for perfume delivery headspaces in packages such as bottles, boxes, bags, etc., in cleaning/drying systems (tumble dryers, dishwashers, dry cleaning systems etc.), in laundry detergents, fabric conditioners, home care products, personal care products (deodorants, anti-perspirants, shampoos, conditioners, cosmetics, skin moisturizers, makeups etc.), in fine fragrances, scented coatings, films, laminates, hygienic articles (fem-care pads, panty liners, diapers, shoe insoles, etc.), in scented inks, scented three dimensional objects, articles for disinfectants delivery, insecticides delivery, insect repellants delivery, flavor delivery, perfume sampling materials etc.

### Example

70 parts of Lavender natural extract are charged to into a vessel (sealed or under reflux) together with 10 parts of Sucrose Acetate Isobutyrate (SAIB from Eastman Chemical) as plasticizer and mixed at room temperature. The temperature is then elevated to 80°C. 10 parts of Pebax 2533 (from Total Fina) as polar thermoplastic elastomer and 10 parts of low melting point polyamide Sylvaclear AF 1900 from Arizona Chemical are charged into the vessel and under stirred till complete dissolution. The composition is then let to cool down and solidify at room temperature. The resulting composition is solid and stable at a temperature of 50°C without bleeding of the volatile material.

## Claims

1. A polymeric composition comprising:
a) a low melting point polyamide polymer
b) a polar thermoplastic elastomer
c) a volatile material.

2. A polymeric composition according to claim 1 wherein the low melting point polyamide polymer has a melting point below 130°C, preferably below 110°C, more preferably below 100°C..

3. A polymeric composition according to any preceding claim wherein the low melting point polyamide polymer is a terminated polyamide, preferably an ester terminated polyamide.

4. A polymeric composition according to any preceding claim wherein the polar thermoplastic elastomer comprises hard phases and soft phases chemically bonded together in the polymer chain.

5. A polymeric composition according to any preceding claim, wherein the polar thermoplastic elastomer comprises at least one polar monomer.

6. A polymeric composition according to any preceding claim wherein the polar thermoplastic elastomer is selected from thermoplastic polyurethanes, thermoplastic polyether ester copolymers and thermoplastic polyether amide copolymers.

7. A polymeric composition according to any preceding claim wherein the low melting point polyamide polymer is from 5% to 40%, preferably from 7% to 30%, more preferably from 15% to 25%, by weight of the polymeric composition, the polar thermoplastic elastomer is from 2% to 30%, preferably from 5% to 20%, more preferably from 7% to 15% by weight of the polymeric composition and the volatile material is from 30% to 90%, preferably from 40% to 80%, more preferably from 50% to 75%, by weight of the polymeric composition.

8. A polymeric composition according to any preceding claim wherein the volatile material is a perfume.

9. A polymeric composition according to claim 8 wherein the perfume comprises an aldehyde, a ketone, an alcohol, a terpene or an ester.

10. A polymeric composition according to any preceding claim which comprises a plasticizer.

11. A polymeric composition according to claim 10 wherein the plasticizer is resent at a level from 0% to 60%, preferably from 5% to 40%, more preferably from 7% to 25%, by weight of the polymeric composition.

12. A polymeric composition according to claim 10 or 11 wherein the plasticizer is selected form the group consisting of citric acid esters, low molecular weight polyesters, polyethers, liquid rosin esters, aromatic sulfonamides, phthalates, benzoates, sucrose esters, derivatives of polyfunctional alcohols (where polyfunctional means having 2 or more hydroxyl groups), adipates, tartrates, sebacates, esters of phosphoric acid, fatty acids and diacids, fatty alcohols and diols, epoxidised vegetable oils etc, and mixtures thereof.
